# EUROPÄISCHE PATENTSCHRIFT

(11) **EP 0 370 220 B1**
(45) Veröffentlichungstag und Bekanntmachung des Hinweises auf die Patenterteilung: **10.01.1996**
(21) Anmeldenummer: 89118855.9
(22) Anmeldetag: 11.10.1989
(51) Int. Cl.: A61K 31/565

(54) **Mittel zur transdermalen Applikation enthaltend Gestoden**
Gestode composition for transdermal application
Composition de Gestoden à application transdermique

(30) Priorität: 27.10.1988 DE 3836862; 29.03.1989 DE 3910578
(43) Veröffentlichungstag der Anmeldung: 30.05.1990
(73) Patentinhaber: SCHERING AKTIENGESELLSCHAFT, D-13353 Berlin (DE)
(72) Erfinder: Günther, Clemens, D-1000 Berlin 21 (DE); Täuber, Ulrich, Dr., D-1000 Berlin 28 (DE); Schmidt-Gollwitzer, Karin, Dr., D-1000 Berlin 39 (DE); Riedl, Jutta, Dr., D-1000 Berlin 12 (DE); Tack, Johannes-Wilhelm, Dr., D-1000 Berlin 20 (DE)

(56) Entgegenhaltungen:
- EP-A- 0 137 278
- EP-A- 0 275 716
- EP-A- 0 279 977
- EP-A- 0 285 563
- STN FILE SERVER INTERNATIONAL, KARLSRUHE, FILE CA & CHEMICAL ABSTRACTS, Band 107, Nr. 13, 1987, Zusammenfassung Nr. 109601a; K. FOTHERBY et al.: "Metabolic investigations with Femodene - an oral contraceptive containing gestodene and ethinylestradiol"
- L.Lange et al: Konzepte in der Humanpharmakologie, Springer Verlag Berlin, 1993, Seiten 65-79

## Beschreibung

Die Erfindung betrifft ein Mittel zur transdermalen Applikation, dadurch gekennzeichnet, daß es Gestoden gegebenenfalls in Kombination mit einem oder mehreren Ostrogen(en) enthalt mit Ausnahme von Mitteln zur transdermalen Applikation, in denen Gestoden zu mindestens 50 % in einem nicht fließfähigen, physiologisch unbedenklichen Gel gelöst ist, welches in einem vernetzten Siliconelastomeren mikrodispers verteilt ist.

Gestoden (13-Ethyl-17β-hydroxy-18,19-dinor-17α-pregna-4,15-dien-3-on), eine Verbindung der Formel 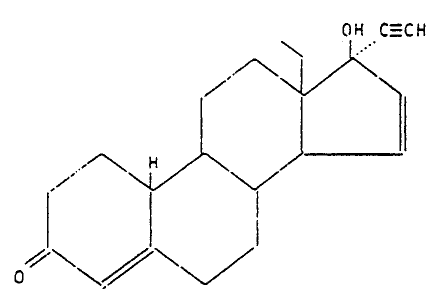
ist bekanntlich eine pharmakologisch wirksame Substanz mit einer außergewohnlich starken gestagenen Wirksamkeit, welche in Kombination mit ostrogen wirksamen Verbindungen zur Herstellung von oral zu applizierenden Mitteln konzeptionsverhindernder Wirkung (Femovan®) verwendet wird.

Es wurde nun gefunden, daß Gestoden gegebenenfalls in Kombination mit einem oder mehreren Ostrogen(en) sehr gut zur Herstellung eines Mittels für die transdermale Applikation des Wirkstoffes oder Wirkstoffgemisches verwendet werden kann.

Transdermal Zu applizierende Arzneimittel haben bekanntlich den Vorzug. daß sie uber einer largeren Zeitraum hin eine gleichmaßigere Freisetzung des Wirkstoffs ermöglichen, als dies in der Regel bei anders - wie zum Beispiel oral -zu applizierenden Mitteln moglich ist. Diese Eigenschaften lassen sich in einer Reihe von endokrinen Erkrankungen vorteilhaft ausnutzen. Für in Wasser schwer lösliche Steroidhormone, wie zum Beispiel die Gestagene ist es aber in der Regel recht problematisch, transdermale Systeme zu erstellen, die eine zur Therapie ausreichende Penetration des Wirkstoffs durch die Haut gewahrleisten.

Es wurde nun gefunden. daß es mit Hilfe des erfindungsgemaßen Mittels uberraschenderweise moglich ist, eine therapeutisch ausreichende sehr gleichmaßige Penetrationsgeschwindigkeit der Steroidhormone durch die Haut zu erzielen. wahrend dies bei den bekannten Steroidhormone enthaltenden transdermal zu applizierenden Mitteln nur bedingt möglich ist. (EP-A 137278 und EP-A 275716)

Geeignete Ostrogene fur das erfindungsgemäße Mittel sind beispielsweise das Estradiol, das Estriol, das Ethinylestradiol und deren Ester (EP-A 163596) wie das Estradiol-dipropionat, das Estradiol-dihexanoat und das Estradiol-didecanoat. Die erfindungsgemäßen Kombinationspräparate enthalten neben Gestoden vorzugsweise 1 bis 3 - insbesondere 1 bis 2 Ostogen(e).

Zur Herstellung pharmazeutischer Präparate kann der Wirkstoff oder das Wirkstoffgemisch in geeigneten fluchtigen Lösungsmitteln und/oder penetrationsverstarkenden Mitteln gelost oder suspendiert werden. Die erhaltenen Losungen oder Suspensionen konnen mit den ublichen Hilfsstoffen, wie Matrixbildnern und Baxteriziden versetzt und gegebenenfalls nach Sterilisation in übliche Dosierbehaltnisse abgefüllt werden. Andererseits ist es aber auch möglich, diese Lösungen oder Suspensionen unter Einbeziehung von Emulgatoren und Wasser zu Lotionen oder Salben weiterzuverarbeiten. Man kann auch - gegebenenfalls unter Zugabe von Treibgas - Sprays herstellen, die in den üblichen Dosierbehältnissen abgefüllt werden konnen.

Geeignete fluchtige Losungsmittel sind beispielsweise niedere Alkohole, Ketone oder niedere Carbonsaureester wie Ethanol, Isopropanol,Aceton oder Ethylacetat, polare Ether, wie Tetrahydrofuran, niedere Kohlenwasserstoffe, wie Cyclohexan oder Benzin oder auch Halogenkohlenwasserstoffe, wie Dichlormethan, Trichlormethan, Trichlortrifluorethan und Trichlorfluormethan. Es bedarf keiner Erläuterung, daß auch Gemische dieser Lösungsmittel geeignet sind.

Geeignete penetrationsverstärkende Mittel sind beispielsweise Alkohole wie 1,2-Propandiol oder Benzylalkohol, gesättigte und ungesättigte Fettalkohole mit 8 bis 18 Kohlenstoffatomen, wie Laurylalkohol oder Cetylalkohol, Kohlenwasserstoffe, wie Mineralol, gesättigte und ungesättigte Fettsauren mit 8 bis 18 Kohlenstoffatomen, wie Stearinsaure oder Olsäure. Fettsaureester der allgemeinen Formel

CH₃-(CH₂)ₙ-COOR

worin
n eine Zahl von 8 bis 18 und
R einen Alkylrest mit maximal 6 Kohlenstoffatomen bedeuten,
oder Dicarbonsaurediester der allgemeinen Formel

R'OCO(CH₂)ₘCOOR'

worin
m eine Zahl von 4 bis 8 und
R' jeweils einen Alkylrest mit maximal 6 Kohlenstoffatomen bedeuten, Fettsäureester, die sich für das erfindungsgemäße Mittel eignen, sind beispielsweise solche der Laurinsäure, Myristinsäure, Stearinsäure und Palmitinsäure, wie zum Beispiel die Methylester, Ethylester, Propylester, Isopropylester, Butylester, sec.-Butylester, Isobutylester dieser Sauren. Besonders bevorzugte Ester sind solche der Myristinsäure, wie deren Methylester und insbesondere deren Isopropylester. Geeeignete Dicarbonsaurediester sind beispielsweise beispielsweise das Diisopropyladipat, Diisobutyladipat und Diisopropylsebacat. Es bedarf keiner naheren Erläuterung, das auch Gemische dieser penetrationsverstarkenden Mittel zur Herstellung des erfindungsgemaßen Mittels geeignet sind.

Die Konzentration, in welcher der Wirkstoff- oder das Wirkstoffgemisch optimalerweise in dem Losungsmittel gelöst oder suspendiert werden, betragt fur Gestoden ublicherweise 0,01 bis 25 Gewichtsprozent. Bei den Ostrogenen ist die Konzentration naturgemäß von der Art des verwendeten Wirkstoffs und der angestrebten Einzeldosis abhängig, sie muß im Einzelfalle mittels der dem Fachmann geläufigen Vorversuche, wie zum Beispiel der Bestimmung der erreichbaren Blutplasmakonzentrationen an Wirkstoff, bei ausgewählten erfindungsgemäßen Mitteln ermittelt werden. Im allgemeinen werden auch hier Wirkstoffkonzentrationen von 0.01 bis 25 Gewichtsprozent Ostrogen im erfindungsgemäßen Mittel ausreichend sein. Das Gewichtsverhaltnis von Gestoden zu dem oder den Ostrogen(en) liegt bei den Kombinationspraparaten bei 5:1 bis 1:10.

Die Bestimmung des Ausmaßes der Geschwindigkeit der percutanen Resorption durch die erfindungsgemäßen Mittel kann beispielsweise mittels radioaktiv markierter Steroidhormone erfolgen.

Frisch bereitete, von subcutanem Fett befreite Haut vom Abdomen haarloser Mause wird in eine Franz Diffusionszelle eingespannt, die als Auffangflüssigkeit isotonische Polyethylenglykol-(MG 400)-Lösung oder Phosphat-Puffer-Losung vom pH 7 enthalt. Dann gibt man 2 µl Testlösung auf die Haut und ermittelt nach 2-, 48 und 72 Stunden mittels Flussigkeits-Scintilationszählung den Gehalt der in die Auffangflüssigkeit gelangten Steroidhormons.

Getestet wurde eine 2 gewichtsprozentige Lösung von Gestoden in Isopropylmyristat (Versuch A) und Paraffin (Versuch B).

Die nachfolgende Tabelle 1 zeigt die in diesem Versuch erhaltenen Ergebnisse:

**TABELLE 1**

| Percutaner Fluß in ng Gestoden pro cm² Hautoberflache und Stunde | | |
|---|---|---|
| Zeitintervall | Versuch A Percutaner Fluß | Versuch B Percutaner Fluß |
| 00 - 24 h | 546 | 755 |
| 24 - 48 h | 379 | 245 |
| 48 - 72 h | 287 | 100 |

Es kann ferner gezeigt werden, daß Gestoden in nicht radioaktiv markierter Form, gelost in 1,2-Propandiol (Versuch C) oder oder in Isopropylmyristat (Versuch D) bei postmenopausalen Frauen eine ausreichende percutane Resorption zeigt.

Getestet werden 0,4 mg Gestoden, gelöst in jeweils 200 µl des entsprechenden Vehikels, die für 48 Stunden auf einer Hautfläche von 10 cm² appliziert werden.

Die nachfolgende Tabelle 2 zeigt die in diesem Versuch erhaltenen Ergebnisse:

**Tabelle 2**

| Percutaner Fluß in ng Gestoden pro cm² Hautoberfläche und Stunde | | |
|---|---|---|
| | Steady state Plasmaspiegel pg/ml | Percutaner Fluß ng/cm²/Std. bei bei 24-48 Std. |
| Versuch C | 250 | 43 |
| Versuch D | 337 | 57 |

Eine noch gleichmaßigere Applikation mit eingestellter Dosierung des Wirkstoffes oder Wirkstoffgemisches kann man erzielen wenn man der Wirkstoff oder das Gemisch in ein transdermales therapeutisches System (TTS) einbettet. Geeignete transdermale therapeutische Systeme sind solche, die man üblicherweise zur percutanen Applikation von Wirkstoffen anwendet (Yie W. Chien: "Tansdermal Controlled Systemic Medications", Marcel Dekker, Inc., New York and Basel, 1987, Dr. Richard Baker: "Analysis of Transdermal Drug Delivery Patents 1934 to 1984" und Analysis of Recent Transdermal Delivery Patents, 1984 - 1986 and Enhancers" Membrane Technology & Reserch 1030 Hamilton Court Menlo Park CA 94025 (415) 328-2228).

So kann man beispielsweise ein solches transdermales therapeutisches System verwenden, welches aus
a) einer undurchlässigen Deckschicht,
   einer an der Deckschicht haftenden, das Gestoden,
   gegebenenfalls das oder die Ostrogen(e) und gewünschtenfalls penetrationsverstarkende Mittel enthaltenden, für diese Komponenten durchlässigen Arzneimittelschicht, die selbsthaftend ist oder von einem Hauthaftkleber abgedeckt oder umgeben ist, welcher ebenfalls penetrationsverstärkende Mittel enthalten kann und
   einer abziehbaren Schutzschicht, oder
b) einer undurchlässigen Deckschicht,
   einem an oder in der Deckschicht befindlichen, das Gestoden, gegebenenfalls das oder die Ostrogen(e) und gewünschtenfalls penetrationsverstärkende Mittel enthaltenden Arzneimittelreservoir,
   für diese Komponenten durchlässigen Polymerschicht,
   einer durchlässigen gegebenenfalls penetrationsverstärkende Mittel enthaltendé Hauthaftkleberschicht und
   einer abziehbaren Schutzschicht besteht.

Ein transdermales therapeutisches System gemaß Variante a stellt ein einfaches Matrixsystem dar. Es kann beispielsweise wie folgt hergestellt werden.

Eine Lösung oder Suspension von 1 bis 25 Gew. % Wirkstoff oder Wirkstoffgemisch, 0-40 Gew. % eines penetrationsverstärkenden Mittels, 30-70 Gew. % eines medizinisch üblichen Klebers aufgefüllt mit einem geeigneten fluchtigen Losungsmittels zu 100 Gew. % wird auf eine plane undurchlässige Deckschicht gestrichen und nach dem Trocknen mit einer abziehbaren Schutzschicht versehen.

Verwendet man einen medizinisch üblichen Matrixbildner, der nach dem Trocknen des Systems nicht oder nicht ausreichend auf der Haut haftet, so kann man das System vor dem Aufbringen der abziehbaren Schutzschicht noch zusatzlich mit einem Hauthaftkleber abdecken oder umgeben.

Geeignete Losungsmittel und penetrationsverstarkenden Mittel sind beispielsweise die bereits erwahnten Flüssigkeiten dieser Art. Als medizinisch übliche Kleber eignen sich beispielsweise Polyacrylate, Silicone, Polyurethane, sowie natürliche oder synthetische Kautschuke. Als weitere Matrixbildner kommen Celluloseether, Polyvinylverbindungen oder Silikate in Betracht.

Als Schutzschichten eignen sich alle Folien, die man üblicherweise bei transdermalen therapeutischen Systemen anwendet. Solche Folien sind beispielsweise silikonisiert oder fluorpolymerbeschichtet.

Als Deckschicht kann man bei diesem System beispielsweise 10 bis 100 µm dicke Folien aus Polyethylen oder Polyester wahlweise pigmentiert oder metallisiert verwenden. Die hierauf aufgebrachte Arzneimittelschicht hat vorzugsweise eine Dicke von 20 bis 500 µm. Die Abgabe der Wirkstoffe erfolgt vorzugsweise uber eine Flache von 5 bis 100 cm².

Ein transdermales therapeutisches System gemäß Variante b kann beispielsweise wie folgt hergestellt werden.

Eine undurchlässige Folie wird durch Warme und/oder Zug so verformt, daß eine 0,1 bis 3 ml fassende Ausbuchtung entsteht. Diese wird mit einer wirkstoffhaltigen Lösung oder Suspension enthaltend 1-50 Gew. % Wirkstoff oder Wirkstoffgemisch in einem penetrationsverstarkenden Mittel gefüllt. Die wirkstoffhaltige Losung oder Suspension kann auch mit bis zu 10 Gew. % Matrixbildner verdickt sein.

Als Abdeckung des Reservoirs zur Haut hin dient eine aufgeschweißte oder aufgeklebte durchlassige Polymerschicht, auf welche eine durchlassige Hauthaftkleberschicht und eine abziehbare Schutzschicht angebracht wird.

Es können bei diesem System die oben erwähnten penetrationsverstarkenden Mittel angewendet werden. Als durchlässige Polymerschicht wird beispielsweise eine 20 bis 200 µm dicke Folie aus Celluloseestern, Celluloseethern, Siliconen oder Polyolefinverbindungen verwendet. Durch Varitation dieser Polymerschicht läßt sich die Diffusionsgeschwindigkeit des Wirkstoffes oder Wirkstoffgemisches innerhalb weiter Grenzen variieren.

Als Kleber und Schutzschicht eignen sich die gleichen Materialien, die bei dem transdermalen therapeutischen System gemäß Variante a beschrieben sind.

So lassen sich durch einfache Variation der verschiedenen Parameter transdermale therapeutische Systeme mit unterschiedlichen Freisetzungsraten des Wirkstoffes oder Wirkstoffgemisches herstellen, die zwecks Lagerung beispelsweise in Aluminiumfolien verpackt werden können.

Es wurde bereits erwahnt, daß das erfindungsgemäße Mittel zur transdermalen Applikation auch als Lotion, Salbe oder als Spray zubereitet sein kann. Derartige Zubereitungen sind in den Fallen von Vorteil, wo keine Okklusion gewunscht wird.

Ein Emulsionsgel zur transdermalen Applikation besteht beispielsweise aus dem Wirkstoff oder Wirkstoffgemisch, penetrationsverstarkenden Mitteln, Emulgatoren (wobei ambiphile Vertreter der penetrationsverstärkenden Mittel als Emulgatoren dienen können) und gegebenenfalls Matrixbildnern. Eine typische Rezeptur besteht aus 0,1-25 Gew. % Wirkstoff oder Wirkstoffgemisch, 0-10 Gew. % Emulgator 0-5 Gew. % Matrixbildner, 0 bis 50 Gew. % penetrationsverstärkenden Mitteln und Wasser zu 100 Gew. %. Das Mittel wird in üblicher Weise emulgiert, und erforderlichenfalls mit den ublichen Antioxidantien, Konservierungsstoffen etc. versetzt.

Einphasige Gele erhalt man beispielsweise durch Losen oder Suspendieren des Wirkstoffs oder das Wirkstoffgemisches in Lösungsmitteln wie Wasser, niederen Alkoholen oder Mischungen derselben, gegebenenfalls unter Zusatz von penetrationsverstarkende Mittel und Verdicken mit Matrixbildnern.

Typische Rezepturen für solche Gele enthalten 0,01-25 Gew. % Wirkstoff oder Wirkstoffgemisch, 1-20 Gew. % Matrixbildner, 0 bis 40 Gew. % penetrationsverstarkenden Mitteln erganzt mit dem Lösungsmittel zu 100 Gew. % .

Auch diese Gele können gewunschtenfalls Antioxidantien, Konservierungsstoffe etc. enthalten.

Eine typische Sprayrezeptur ist beispelsweise folgende:
1-25 Gew. % Wirkstoff oder Wirkstoffgemisch, 0-20 Gew. % Matrixbildner, 0-60 Gew. % penetrationsverstärkenden Mitteln ergänzt mit Lösungsmitteln und gegebenenfalls Treibmitteln zu 100 %. Verwendet man Druckgaspackungen, so kann das Treibmittel entfallen.

Die erfindungsgemäßen gestodenhaltigen Mittel zur transdermalen Applikation können zur Behandlung der gleichen Erkrankungen angewendet werden, wie die vorbekannten, beispielsweise oral zu applizierenden Mittel die hochwirksame Gestagene enthalten. Darüberhinaus konnen die erfindungsgemäßen gegebenenfalls östrogenhaltigen Präparate auch zur Konzeptionsverhütung Anwendung finden. Besondere Vorteile haben die erfindungsgemäßen Mittel bei der Behandlung von Erkrankungen, die eine Langzeitbehandlung mit relativ hoher Dosierung der Wirkstoffe erfordern. Hier kann die Applikationsfrequenz wesentlich verringert werden und ein wesentlich gleichmaßiger Blutplasmaspiegel erzielt werden. Vorteilhaft ist ferner. daß gastrointestinale Nebenwirkungen nicht zu erwarten sind und bei östrogenhaltigen Kombinationspräparaten die erste Leberpassage umgangen wird und daß die Dosis an Ostrogen vermindert werden kann.

Diese Vorteile lassen die östrogenfreie Monotherapeutika der vorliegenden Erfindung als besonders geeignet erscheinen um beispielsweise die Endometriose, gestagenabhangige Tumore benigne Brusterkrankungen oder das pramenstruelle Syndrom zu behandeln.

Die transdermale Anwendung von Estrogenen in sequentieller oder kontinuierlicher Kombination mit Gestoden bietet besondere Vorteile, beispielsweise zur Behandlung klimakterischer Beschwerden, zur Praventation der Osteoporose, zur Zyklusregulierung und zur Zyklusstabilisation.

Die nachfolgenden Ausführungsbeispiele dienen zur näheren Erlauterung der Erfindung. In ihnen wurden folgende Handelsprodukte verwendet:
Polyesterfolie von 0,074 mm Dicke (SkotchPak® 1009) des Herstellers 3M; Polypropylenfolie (Celgard® 2500) des Herstellers Celanese, Linerfolie SkotchPak® 1022 und 1360 vom Hersteller 3M; Transferkleber 9871 vom Hersteller 3M, Polyacrylester-Kleber vom Typ Sichello® J 6610-21 des Herstellers Henkel KG, Silikonklebstoff vom Typ X-7-2960 des Herstellers Dow Corning und Hydroxypropylcellulose des Typs Klucel® HXF des Herstellers Hercules.

### Beispiel 1

In 62,4 g einer 50 %igen Losung von Silikonklebstoff in Benzin werden nacheinander
- 0,8 g: Gestoden und
- 8,0 g: 1,2-Propandiol
unter Rühren gelost bzw. suspendiert (da die Klebstoffe trübe sind, laßt sich nicht klar entscheiden, ob eine vollständige Lösung vorliegt). Nach Entgasen des Ansatzes wird die Losung/Suspension mittels einer Beschichtungsvorrichtung auf Polyesterfolie in der Weise aufgetragen, daß nach Entfernung des fluchtigen Losemittels ein gleichmaßiger Film von 40 g/m² Feststoffauftrag entsteht. Anschließend wird mit einem fluorpolymerbeschichteten Polyester-Liner kaschiert. Das so erhaltene Laminat wird mittels einer Stanzvorrichtung in Einzelpflaster von 10 cm² Flache geteilt und in Aluminiumfolie verpackt. Das Pflaster haftet nach Abziehen der Liner-Folie auf der Haut.

Die Gehaltsbestimmung ergibt eine gleichmäßige Wirkstoffverteilung von 0,08 mg/cm² im Mittel. Das Pflaster wird ferner über ihr in-vitro-Freigabeverhalten in Wasser uber 30 Stunden bei 32° C charakterisiert. Aus einem typischen Matrixfreigabeverlauf errechnet sich nach Linearisierung eine Freisetzungsgeschwindigkeit fur Gestoden von 0,4 µg/cm²/h.

### Beispiel 2

In 170 g einer 50 %igen Lösung von Polyacrylester-Klebstoff in Aceton/Benzin werden nacheinander
- 5,0 g: Gestoden und
- 10,0 g: Isopropylmyristat
unter Ruhren gelost bzw. suspendiert. Nach Entgasen des Ansatzes wird die Lösung/Suspension mittels einer Beschichtungsvorrichtung auf Polyesterfolie in der Weise aufgetragen, daß nach Entfernung der fluchtigen Losemittel ein gleichmäßiger Film von 160 g/m² Feststoffauftrag entsteht, Anschließend wird mit einer silikonisierten wirkstofffreien Liner-Folie kaschiert. Das so erhaltene Laminat wird mittels einer Stanzvorrichtung in Einzelpflaster von 10 cm² Flache geteilt und in Aluminiumfolie verpackt. Das Pflaster haftet nach Abziehen der Liner-Folie auf der Haut.

Der Gehalt an Gestoden beträgt im Mittel 0,5 mg/cm². Die mittlere Freigabegeschwindigkeit von Gestoden liegt bei 0,3 µg/cm²/h.

### Beispiel 3

Zu 112 g einer 50 %igen Lösung von Polyacrylester-Klebstoff in Aceton/Benzin werden nacheinander
- 3,5 g: Estradiol
- 3,5 g: Gestoden und
- 7,0 g: 1,2-Propandiol mit 10 % 1-Dodecanol
unter Ruhren gelost bzw. suspendiert. Nach Entgasen des Ansatzes wird die Lösung/Suspension mittels einer Beschichtungsvorrichtung auf Polyesterfolie in der Weise aufgetragen, daß nach Entfernung der flüchtigen Lösemittel ein gleichmäßiger Film von 70 g/m² Feststoffauftrag entsteht. Anschließend wird mit einer silikonisierten wirkstofffreien Liner-Folie kaschiert. Das so erhaltene Laminat wird mittels einer Stanzvorrichtung in Einzelpflaster von 10 cm² Flache geteilt und in Aluminiumfolie verpackt. Das Pflaster haftet nah Abziehen der Liner-Folie auf der Haut.

Der Gehalt an Estradiol und Gestoden liegt gleichermaßen bei je 0,35 mg/cm².

Aus diesem Pflaster werden innerhalb von 48 Stunden im in-vitro Test in Wasser von 32° C mit 0,6 µg/cm²/h fur Estradiol höhere Freigaberaten erzielt als fur Gestoden, wo der Wert 0,4 µg/cm²/h beträgt.

### Beispiel 4

Eine Polyesterfolie von 7,4 cm Durchmesser wird mittels Zug und Warme so verformt, daß eine runde Ausbuchtung von 10 cm² Fläche entsteht. Diese wird mit 1 ml einer Suspension von
- 2,5 mg: Estradiol und
- 2,5 mg: Gestoden
in 1,2-Propandiol, welches 10 % Laurinsaure enthält, gefüllt. Eine Polypropylen- oder Celluloseacetatbutyrat-Folie wird am Rand aufgeschweißt. Je nach Druck pro Zeiteinheit liegt die Siegeltemperatur zwischen 70° C und 100° C. Auf die durchlassige Polymerschicht wird Haftkleberfolie transferiert. Das Pflaster wird mit einem Liner versehen und in Aluminiumfolie verpackt.

Aus diesem Pflaster werden fur beide Wirkstoffe gleichermaßen in-vitro-Freigaberaten in Wasser von 32° C zwischen 0,02 bis 0,08 µg/cm²/h erzielt.

### Beispiel 5

In 76,78 g Ethanol (96 Vol %ig) oder Isopropanol werden nacheinander
- 0,2 g: Estradiol
- 0,02 g: Gestoden
- 10,0 g: 1,2-Propandiol und
- 10,0 g: Isopropylmyristat
gelöst. Dann setzt man der Lösung 3 g Hydroxypropylcellulose zu und ertfernt aus ihr die Luft. Nach 2 Stunden Quellzeit wird das Gel in Aluminiumtuben mit dreifacher Innenschutzlackierung abgefüllt.

Die Gehaltsbestimmung ergibt eine homogene Wirkstoffverteilung im Gel mit Werten von 95 % bei 105 % des Sollwertes.

### Beispiel 6

20,00 g Gestoden werden in 1000 g Isopropylmyristat gelost, sterilfiltriert und unter aseptischen Bedingungen in Arzneiflaschen a 5 ml abgefullt.

## Patentansprüche

1. Mittel zur transdermalen Applikation, dadurch gekennzeichnet, daß es Gestoden gegebenenfalls in Kombination mit einen oder mehreren Ostrogen(en) enthält mit Ausnahme von Mitteln zur transdermalen Applikation, in denen Gestoden zu mindestens 50 % in einem nicht fließfähigen, physiologisch unbedenklichen Gel gelöst ist, welches in einem vernetzten Siliconelastomeren mikrodispers verteilt ist.

2. Mittel zur transdermalen Applikation gemäß Patentanspruch 1, dadurch gekennzeichnet, daß als Ostrogen(e) Estradiol, Estriol, 17α-Ethinylestradiol oder Ester dieser Verbindungen verwendet werden.

3. Mittel zur transdermalen Applikation gemäß Patentanspruch 1 und 2, dadurch gekennzeichnet, daß das Mittel ein transdermales therapeutisches System (TTS) ist.

4. Mittel zur transdermalen Applikation gemäß Patentanspruch 3, dadurch gekennzeichnet, daß das transdermale therapeutische System aus
a) einer undurchlässigen Deckschicht,
einer an der Deckschicht haftenden, das Gestoden,
gegebenenfalls das oder die Ostrogen(e) und gewünschtenfalls penetrationsverstärkende Mittel enthaltenden, für diese Komponenten durchlassigen Arzneimittelschicht, die selbsthaftend ist oder von einem Hauthaftkleber abgedeckt oder umgeben ist, welcher ebenfalls penetrationsverstarkende Mittel enthalten kann und
einer abziehbaren Schutzschicht, oder
b) einer undurchlässigen Deckschicht,
einem an oder in der Deckschicht befindlichen, das Gestoden, gegebenenfalls das oder die Ostrogen(e) und gewünschtenfalls penetrationsverstärkende Mittel enthaltenden Arzneimittelreservoir,
einer fur diese Komponenten durchlässigen Polymerschicht,
einer durchlassigen gegebenenfalls penetrationsverstarkende Mittel enthaltende Hauthaftkleber-Schicht und
einer abziehbaren Schutzschicht besteht.

5. Verwendung von Gestoden gegebenenfalls in Kombination mit einem oder mehrerer Ostrogen(en) zur Herstellung eines Mittels fur die transdermale Applikation des Wirkstoffes oder Wirkstoffgemisches mit Ausnahme von Mitteln zur transdermalen Applikation, in denen Gestoden zu mindestens 50 % in einem nicht fließfähigen, physiologisch unbedenklichen Gel gelöst ist, welches in einem vernetzten Siliconelastomeren mikrodispers verteilt ist.

6. Verwendung von Gestoden in Kombination mit einem oder mehreren Ostrogen(en) zur Herstellung eines Mittels gemäß Patentanpruch 1, dadurch gekennzeichnet, als Ostrogen(e) Estradiol, Estriol, 17α-Ethinylestradiol oder Ester dieser Verbindungen verwendet werden.

7. Verwendung von Gestoden gegebenenfalls in Kombination mit einem oder mehreren Ostrogen(en) zur Herstellung eines Mittels gemaß Patentanspruch 1 und 2, dadurch gekennzeichnet, daß das Mittel ein transdermales therapeutisches System (TTS) ist.

8. Verwendung von Gestoden gegebenenfalls in Kombination mit einem oder mehreren Ostrogen(en) zur Herstellung eines Mittels gemaß Patentanspruch 3, dadurch gekennzeichnet, daß das transdermale therapeutische System aus
a) einer undurchlassigen Deckschicht,
einer an der Deckschicht haftenden, das Gestoden,
gegebenenfalls das oder die Ostrogen(e) und gewunschtenfalls penetrationsverstarkende Mittel enthaltenden, fur diese Komponenten durchlassigen Arzneimittelschicht, die selbsthaftend ist oder von einem Hauthaftkleber abgedeckt oder umgeben ist, welcher ebenfalls penetrationsverstärkende Mittel enthalten kann und
einer abziehbaren Schutzschicht, oder
b) einer undurchlässigen Deckschicht,
einem an oder in der Deckschicht befindlichen, das Gestoden, gegebenenfalls das oder die Ostrogen(e) und gewünschtenfalls penetrationsverstärkende Mittel enthaltenden Arzneimittelreservoir,
einer fur diese Komponenten durchlässigen Polymerschicht.
einer durchlassigen gegebenenfalls penetrationsverstarkende Mittel enthaltende Hauthaftkleber-Schicht und
einer abziehbaren Schlutzschicht besteht.

9. Verwendung von ostrogenfreien Mitteln zur transdermalen Applikation gemaß Patentanspruch 1 bis 4 zur Herstellung von Arzneimitteln zur Behandlung der Endometriose, zur Behandlung von gestagenabhangiger Tumoren und zur Behandlung des pramenstruellen Syndroms.

10. Verwendung von Mitteln zur transdermalen Applikation gemäß Patentanspruch 1 bis 4 gegebenenfalls in Kombination mit östrogenhaltigen Mitteln zur Herstellung von Arzneimitteln zur Behandlung klimakterischer Beschwerden, zur Pravention der Osteoporose, zur Zyklusregulierung und zur Zyklusstabilisation.

## Claims

1. Preparation for transdermal administration, characterised in that it comprises gestoden optionally in combination with one or more oestrogen(s), with the exception of preparations for transdermal administration in which gestoden is dissolved to at least 50 % in a non-flowable, physiologically acceptable gel that is microdispersed in a cross-linked silicone elastomer.

2. Preparation for transdermal administration according to patent claim 1, characterised in that oestradiol, oestriol, 17α-ethynyloestradiol or esters of those compounds are used as the oestrogen(s).

3. Preparation for transdermal administration according to patent claims 1 or 2, characterised in that the preparation is a transdermal therapeutic system (TTS).

4. Preparation for transdermal administration according to patent claim 3, characterised in that the transdermal therapeutic system consists of
a) an impermeable cover layer,
a medicament layer that adheres to the cover layer, comprises the gestoden, optionally the oestrogen(s) and, if desired, penetration promoters and is permeable to those components, and that is self-adhesive or is covered or enclosed by a skin adhesive which may likewise comprise penetration promoters, and
a removable protective layer, or
b) an impermeable cover layer,
a medicament reservoir that is located on or in the cover layer and that comprises the gestoden, optionally the oestrogen(s) and, if desired, penetration promoters,
a polymer layer permeable to those components,
a permeable skin-adhesive layer which optionally comprises penetration promoters, and
a removable protective layer.

5. Use of gestoden optionally in combination with one or more oestrogen(s) in the production of a preparation for the transdermal administration of the active ingredient or active ingredient mixture, with the exception of preparations for transdermal administration in which gestoden is dissolved to at least 50 % in a non-flowable, physiologically acceptable gel that is microdispersed in a cross-linked silicone elastomer.

6. Use of gestoden in combination with one or more oestrogen(s) in the production of a preparation according to patent claim 1, characterised in that oestradiol, oestriol, 17α-ethynyloestradiol or esters of those compounds are used as the oestrogen(s).

7. Use of gestoden optionally in combination with one or more oestrogen(s) in the production of a preparation according to patent claims 1 or 2, characterised in that the preparation is a transdermal therapeutic system (TTS).

8. Use of gestoden optionally in combination with one or more oestrogen(s) in the production of a preparation according to patent claim 3, characterised in that the transdermal therapeutic system consists of
a) an impermeable cover layer,
a medicament layer that adheres to the cover layer, comprises gestoden, optionally the oestrogen(s) and, if desired, penetration promoters and is permeable to those components, and that is self-adhesive or is covered or enclosed by a skin adhesive which may likewise comprise penetration promoters, and
a removable protective layer, or
b) an impermeable cover layer, a medicament reservoir that is located on or in the cover layer and that comprises the gestoden, optionally the oestrogen(s) and, if desired, penetration promoters,
a polymer layer permeable to those components,
a permeable skin-adhesive layer which optionally comprises penetration promoters, and
a removable protective layer.

9. Use of oestrogen-free preparations for transdermal administration according to any one of patent claims 1 to 4 for the production of medicaments for the treatment of endometriosis, for the treatment of gestagen-dependent tumours and for the treatment of premenstrual syndrome.

10. Use of preparations for transdermal administration according to any one of patent claims 1 to 4 optionally in combination with oestrogen-containing preparations for the production of medicaments for the treatment of climacteric disorders, for the prevention of osteoporosis, for regulating the menstrual cycle and for stabilising the menstrual cycle.

## Revendications

1. Produit pour administration transdermique ou percutanée, caractérisé en ce qu'il contient du gestodène, éventuellement en association avec un ou plusieurs oestrogène(s), à l'exclusion de produits pour administration transdermique, dans lesquels le gestodène est dissous en une proportion d'au moins 50 % dans un gel physiologiquement tolérable et non fluidifiable, lequel est réparti en microdispersion dans une silicone élastomère réticulée.

2. Produit pour administration percutanée selon la revendication 1, caractérisé en ce qu'on utilise comme oestrogène(s) l'oestradiol, l'oestriol, le 17-α-éthyniloestradiol ou des esters de ces composés.

3. Produit pour administration percutanée selon la revendication 1 et 2, caractérisé en ce que le produit est un système thérapeutique transdermique ou percutané.

4. Produit pour administration transdermique ou percutanée selon la revendication 3, caractérisé en ce que le système thérapeutique transdermique consiste en :
a) une couche imperméable de couverture,
une couche de médicament, adhérant à la couche de couverture et contenant du gestodène, éventuellement le ou les oestrogène(s) et si on le souhaite un agent amplifiant la pénétration, cette couche étant perméable à ces constituants et étant auto-adhérente ou étant recouverte ou entourée d'une colle d'adhérence à la peau qui peut éventuellement contenir de l'agent amplifiant la pénétration, et
une couche protectrice amovible, ou
b) une couche de couverture imperméable,
un réservoir de médicament se trouvant sur ou dans la couche de couverture et contenant le gestodène, éventuellement le ou les oestrogène(s) et si on le souhaite de l'agent amplifiant la pénétration,
une couche de polymère perméable à ces constituants,
une couche de colle pour adhérence à la peau, couche perméable et contenant éventuellement de l'agent amplifiant la pénétration et,
une couche protectrice amovible.

5. Utilisation de gestodène, éventuellement en combinaison ou association avec un ou plusieurs oestrogène(s) pour préparer un produit pour l'administration transdermique ou percutanée de la substance active ou du mélange de substances actives, à l'exclusion de produits pour une administration transdermique percutanée dans lesquels le gestodène est dissous à 50 % au moins dans un gel physiologiquement tolérable et non fluidifiable, lequel est réparti en microdispersion dans une silicone élastomère réticulée.

6. Utilisation du gestodène en combinaison ou association avec un ou plusieurs oestrogène(s) pour préparer un produit selon la revendication 1, caractérisée en ce qu'on utilise comme oestrogène(s) de l'oestradiol, de l'oestriol, du 17-α-éthynyloestradiol ou des esters de ces composés.

7. Utilisation du gestodène, éventuellement en association avec un ou plusieurs oestrogène(s), pour préparer un produit selon la revendication 1 et 2, caractérisée en ce que le produit est un système thérapeutique transdermique ou percutané.

8. Utilisation du gestodène, éventuellement en association avec un ou plusieurs oestrogène(s) pour préparer un produit selon la revendication 3, utilisation caractérisée en ce que le système thérapeutique transdermique ou percutané consiste en :
a) une couche de couverture imperméable,
une couche de médicament adhérant à la couche de couverture, qui contient du gestodène éventuellement le ou les oestrogène(s) et si on le souhaite de l'agent amplifiant la pénétration, couche perméable à ces constituants, qui est auto-adhérente ou est recouverte ou entourée d'une colle pour adhérence à la peau, laquelle couche contient également un agent amplifiant la pénétration, et
une couche protectrice amovible ou,
b) une couche de couverture imperméable,
un réservoir de médicament se trouvant sur ou dans la couche de couverture, qui contient du gestodène, éventuellement un ou plusieurs oestrogène(s) et, si on le souhaite, de l'agent amplifiant la pénétration,
une couche polymère perméable à ces constituants,
une couche de colle pour adhérence à la peau, perméable et comportant éventuellement de l'agent d'amplification de la pénétration, et
une couche protectrice amovible.

9. Utilisation d'un produit dépourvu d'oestrogène pour une administration transdermique ou percutanée selon les revendications 1 à 4 pour préparer des médicaments pour le traitement d'une endométriose, pour le traitement de tumeurs dépendant des gestagènes et pour le traitement du syndrome prémenstruel.

10. Utilisation de produit pour administration transdermique ou percutanée selon la revendication 1 à 4, éventuellement en combinaison ou association avec des produits contenant de l'oestrogène pour préparer des médicaments pour le traitement d'affections climatiques, pour la prévention de l'ostéoporose, pour la régulation des cycles et pour la stabilisation des cycles (menstruels).
